# EUROPÄISCHE PATENTANMELDUNG

(11) **EP 3 400 974 A1**
(43) Veröffentlichungstag der Anmeldung: **14.11.2018**
(21) Anmeldenummer: 17170710.2
(22) Anmeldetag: 11.05.2017
(51) Int. Cl.: A61M 1/12, A61M 1/10

(54) **HERZPUMPENEINRICHTUNG UND BETRIEBSVERFAHREN FÜR EINE HERZPUMPENEINRICHTUNG**

(71) Anmelder: Berlin Heart GmbH, 12247 Berlin (DE)
(72) Erfinder: Uennigmann, Benno, 10437 Berlin (DE); Frischke, Michael, 15834 Rangsdorf (DE); Peters, Oliver, 14129 Berlin (DE); Jankowsky, Florian, 15848 Friedland (DE)
(74) Vertreter: Pfenning, Meinig & Partner mbB

(57) **Zusammenfassung**

Die Anmeldung betrifft eine Herzpumpeneinrichtung (8) und ein Betriebsverfahren für eine Herzpumpeneinrichtung (8). Die vorgeschlagene Herzpumpeneinrichtung (8) umfasst eine implantierbare Herzpumpe (1) und ein Steuergerät (6) zum Steuern der Herzpumpe (1). Das Steuergerät (6) und die Herzpumpe (1) sind über eine Leitung mit Adern (21, 22, 23) miteinander verbunden. Außerdem ist das Steuergerät (6) eingerichtet, die Herzpumpe (1) über eine erste der Adern (21) mit elektrischer Energie zu versorgen. Zudem weisen das Steuergerät (6) und die Herzpumpe (1) jeweils eine Koppelschnittstelle (13, 16) auf. Hierbei sind über diese Koppelschnittstellen (13, 16) elektrische Signale zur Datenübertragung zwischen dem Steuergerät (6) und der Herzpumpe (1) in die erste Ader (21) einkoppelbar beziehungsweise aus der ersten Ader (21) auskoppelbar.

## Beschreibung

Die vorliegende Anmeldung liegt auf dem Gebiet der Medizintechnik und betrifft eine Herzpumpeneinrichtung. Außerdem betrifft die vorliegende Anmeldung ein Betriebsverfahren für eine Herzpumpeneinrichtung.

Aus dem Stand der Technik sind implantierbare Herzpumpen bekannt. Diese Herzpumpen kommen zum Einsatz, wenn eine Herzfunktion eines Patienten unterstützt oder ersetzt werden muss. Gängige Systeme, die hierbei verwendet werden, sind sogenannte VAD (ventricular assist devices). Derartige Herzpumpen können beispielsweise als sogenanntes LVAD (left ventricular assist device), RVAD (right ventricular assist device) oder BiVAD (bi-ventricular assist device) ausgeführt sein. Diese Systeme umfassen neben der Herzpumpe, die im Betrieb in dem Patienten implantiert ist, in der Regel ein außerhalb eines Körpers des Patienten angeordnetes und mit der Herzpumpe über eine perkutane Leitung (Driveline) verbundenes Steuergerät. Die Driveline verläuft in einem Abschnitt zwischen dem Steuergerät und einer Einstichstelle außerhalb des Körpers des Patienten und in einem Abschnitt zwischen der Einstichstelle und der Herzpumpe innerhalb des Körpers des Patienten. Das Steuergerät kann beispielweise einen integrierten Akkumulator (wiederaufladbare Batterie) aufweisen oder mit einem Akkumulator verbindbar sein, so dass die implantierte Herzpumpe durch das Steuergerät über die perkutane Leitung mit elektrischer Energie versorgt werden kann. Die Herzpumpe umfasst in der Regel einen Motor mit einem Stator und einem mit einer Beschaufelung versehenen Rotor. Durch die von dem Steuergerät gelieferte elektrische Energie kann in der Regel der Motor der Herzpumpe angetrieben werden, indem beispielsweise ein Stromfluss in Wicklungen des Stators erzeugt wird, durch den der Rotor mitsamt Beschaufelung zum Fördern von Blut des Patienten in Drehung versetzt wird. Ein System verwandter Art ist beispielsweise in der Druckschrift EP 3 090 767 A1 beschrieben.

Es ist eine Aufgabe der vorliegenden Anmeldung, eine verbesserte Herzpumpeneinrichtung vorzuschlagen. Insbesondere ist es eine Aufgabe der vorliegenden Anmeldung, eine Herzpumpeneinrichtung vorzuschlagen, durch die ein Wohlbefinden eines Patienten gesteigert und eine Haut- und Gewebeschädigung des Patienten reduziert werden kann, wobei gleichzeitig eine komplexe Steuerungsmöglichkeit der Herzpumpe gewährleistet sein soll.

Diese Aufgabe wird gelöst durch eine Herzpumpeneinrichtung mit den Merkmalen des Anspruchs 1. Vorteilhafte Weiterbildungen ergeben sich mit den Merkmalen der abhängigen Ansprüche und der Ausführungsbeispiele.

Die vorgeschlagene Herzpumpeneinrichtung umfasst eine implantierbare Herzpumpe und ein Steuergerät zum Steuern der Herzpumpe. Das Steuergerät und die Herzpumpe sind über eine Leitung mit Adern miteinander verbunden. Außerdem ist das Steuergerät eingerichtet, die Herzpumpe über eine erste der Adern mit elektrischer Energie zu versorgen. Zudem weisen das Steuergerät und die Herzpumpe jeweils eine Koppelschnittstelle auf. Hierbei sind über diese Koppelschnittstellen elektrische Signale zur Datenübertragung zwischen dem Steuergerät und der Herzpumpe in die erste Ader einkoppelbar beziehungsweise aus der ersten Ader auskoppelbar.

Bei einem Betriebsverfahren für eine wie oben oder unten beschriebene Herzpumpeneinrichtung wird die elektrische Energie von dem Steuergerät zu der Herzpumpe über die erste Ader übertragen. Außerdem wird ein elektrisches Signal in die erste Ader durch die Koppelschnittstelle des Steuergeräts oder die Koppelschnittstelle der Herzpumpe zum Übertragen von Daten zwischen dem Steuergerät und der Herzpumpe eingekoppelt. Das elektrische Signal wird anschließend aus der ersten Ader durch die Koppelschnittstelle der Herzpumpe bzw. die Koppelschnittstelle des Steuergeräts ausgekoppelt.

Die Datenübertragung ermöglicht eine komplexe Steuerung der Herzpumpe, beispielsweise anhand einer Vielzahl von Sensordaten. Die über die elektrischen Signale zwischen dem Steuergerät und der Herzpumpe ausgetauschten Daten können beispielsweise Sensordaten, Patientendaten, Patientenidentifizierungsdaten, Hardwareidentifizierungsdaten, Softwareversionsdaten, Konfigurationsdaten, Betriebsparameter, Fehlerdaten, Informationen zu Warnbedingungen, Messdaten, Zeitinformationen, Rechenergebnisse, Informationen zur Rotorposition und/oder Motordaten sein. Dadurch, dass die Energieversorgung der Herzpumpe sowie die Datenübertragung über dieselbe Ader bzw. dieselben Adern erfolgt, kann die Leitung, über die die Herzpumpe und das Steuergerät miteinander verbunden sind, mit im Vergleich zum Stand der Technik reduziertem Querschnitt ausgebildet sein, da eine geringere Anzahl von Adern erforderlich ist. Somit kann eine Gewebeschädigung eines Patienten reduziert und ein Wohlbefinden des Patienten erhöht werden.

In typischen Ausführungen ist das Steuergerät ein extrakorporales Steuergerät, so dass die Leitung während eines Betriebs der Herzpumpe transkutan verläuft. In diesem Fall ermöglicht der geringe Querschnitt der Leitung, dass eine Einstichstelle in einer Haut des Patienten, durch die die Leitung geführt ist, mit geringem Querschnitt ausgebildet sein kann. Daher kann die vorgeschlagene Herzpumpeneinrichtung zu einer Entlastung dieser Einstichstelle und zu einer Verringerung eines Infektionsrisikos führen.

Die Datenübertragung zwischen dem Steuergerät und der Herzpumpe erfolgt nicht notwendigerweise ausschließlich über die erste Ader. Zudem wird die Herzpumpe nicht notwendigerweise ausschließlich über die erste Ader mit elektrischer Energie versorgt. Es kann hingegen vorgesehen sein, dass die Datenübertragung bzw. die Energieversorgung durch ein Zusammenspiel der ersten Ader mit weiteren Adern ermöglicht wird. In typischen Ausführungen weist die Leitung eine zweite Ader und/oder eine dritte Ader auf. Die Herzpumpe kann in diesem Fall über die erste Ader und die zweite Ader und gegebenenfalls die dritte Ader mit elektrischer Energie versorgbar sein. Es kann ferner vorgesehen sein, dass über die Koppelschnittstellen des Steuergeräts und der Herzpumpe weitere elektrische Signale zur Datenübertragung zwischen dem Steuergerät und der Herzpumpe in die zweite Ader und/oder die dritte Ader einkoppelbar beziehungsweise aus der zweiten bzw. dritten Ader auskoppelbar sind. Die Leitung weist in der Regel einen Mantel auf, der sämtliche Adern der Leitung umgibt. Dieser Mantel kann aus einem biokompatiblen Material gefertigt sein und beispielsweise einen Kunststoff, insbesondere PU oder Silikon, enthalten.

Das Steuergerät und die Herzpumpe können zum Übertragen von Daten von der Herzpumpe zu dem Steuergerät über die Koppelschnittstellen und über die erste, die zweite und/oder die dritte Ader eingerichtet sein. In diesem Fall sendet die Herzpumpe die elektrischen Signale bzw. Daten, die anschließend von dem Steuergerät empfangen werden. Es kann beispielsweise vorgesehen sein, dass die Herzpumpe Sensoren aufweist. Von den Sensoren erfasste Sensordaten können dann beispielsweise in oben und/oder unten beschriebener Weise von der Herzpumpe zum Steuergerät übertragen werden.

Es kann zusätzlich oder alternativ außerdem vorgesehen sein, dass das Steuergerät und die Herzpumpe zum Übertragen von Daten von dem Steuergerät zu der Herzpumpe über die Koppelschnittstellen und über die erste, die zweite und/oder die dritte Ader eingerichtet sind. In diesem Fall sendet das Steuergerät die elektrischen Signale bzw. Daten, die anschließend von der Herzpumpe empfangen werden. Es ist in der Regel vorgesehen, dass das Steuergerät und die Herzpumpe jeweils eingerichtet sind, die elektrischen Signale bzw. Daten sowohl zu senden als auch zu empfangen. In typischen Ausführungen sind das Steuergerät und die Herzpumpe eingerichtet, zeitgleich elektrische Signale bzw. Daten in beiden Richtungen im Sinne eines Gegenbetriebs (Full-Duplex-Betrieb) zu übertragen. Es sind aber auch beispielsweise eine gleichzeitige Übertragung über zwei Übertragungswege oder ein Halb-Duplex-Betrieb möglich.

Das Steuergerät kann einen mit der Koppelschnittstelle des Steuergeräts verbundenen Modulator aufweisen. Der Modulator des Steuergeräts kann eingerichtet sein, ein Trägerfrequenzsignal zum Erzeugen der in die erste, die zweite und/oder die dritte Ader einzukoppelnden elektrischen Signale zur Datenübertragung an die Herzpumpe zu modulieren. In weiteren Ausführungen weist die Herzpumpe einen mit der Koppelschnittstelle der Herzpumpe verbundenen Modulator auf. Der Modulator der Herzpumpe kann eingerichtet sein, ein Trägerfrequenzsignal zum Erzeugen der in die erste, die zweite und/oder die dritte Ader einzukoppelnden elektrischen Signale zur Datenübertragung an das Steuergerät zu modulieren. Der Modulator des Steuergeräts beziehungsweise der Herzpumpe empfängt die Daten beispielsweise als digitale elektrische Signale. Die empfangende Herzpumpe bzw. das empfangende Steuergerät weist in der Regel einen Demodulator auf, durch den die empfangenen elektrischen Signale demoduliert werden. In der Regel werden die elektrischen Signale hierbei in digitale Signale umgewandelt. Anschließend werden die empfangenen und demodulierten Daten typischerweise an einen Mikrocontroller der Herzpumpe bzw. der Steuereinheit übertragen und gegebenenfalls weiterverarbeitet. Die durch den Modulator der Herzpumpe und/oder des Steuergeräts erzeugte Modulation auf die Trägerfrequenz kann beispielsweise durch ein an sich bekanntes digitales Modulationsverfahren erfolgen, beispielsweise durch eine Amplitudenmodulation (amplitude-shift keying, ASK), eine Frequenzmodulation (frequency-shift keying), eine Phasenmodulation (phase-shift keying) oder durch Mischformen davon. Insbesondere Bauteile, die ein ASK-Verfahren zur Modulation bzw. Demodulation verwenden, lassen sich besonders einfach und platzsparend implementieren. Die Modulatoren und/oder Demodulatoren werden in typischen Ausführungen durch entsprechend ausgelegte Schaltkreise gebildet, können aber auch softwarebasiert implementiert sein.

Das Steuergerät kann eingerichtet sein, ein Trägerfrequenzsignal über die Koppelschnittstelle des Steuergeräts in eine Ader oder mehrere der Adern der Leitung, beispielsweise in die erste, die zweite und/oder die dritte Ader einzukoppeln. Die Herzpumpe ist in einigen Ausführungen eingerichtet, das Trägerfrequenzsignal über die Koppelschnittstelle der Herzpumpe auszukoppeln. Der Modulator der Herzpumpe kann ferner eingerichtet sein, das Trägerfrequenzsignal zum Erzeugen der in die erste, die zweite und/oder die dritte Ader einzukoppelnden elektrischen Signale zur Datenübertragung an die Herzpumpe zu modulieren. Auf diese Weise wird eine Datenübertragung von der Herzpumpe an das Steuergerät mittels modulierter Trägerfrequenz ermöglicht, ohne dass eine Erzeugung der Trägerfrequenz in der Herzpumpe notwendig ist. Dadurch kann ein Bauteilaufwand auf Seiten der implantierbaren Herzpumpe gering gehalten werden, und diese kann ferner besonders kompakt ausgebildet sein. Falls zugleich eine Datenübertragung von dem Steuergerät zu der Herzpumpe erfolgt, dann wird durch den Modulator der Steuereinheit in der Regel eine andere Trägerfrequenz verwendet, so dass Störungen aufgrund eines Übersprechens zwischen den beiden Übertragungsrichtungen vermieden werden.

Zur Versorgung der Herzpumpe mit elektrischer Energie sind die erste, die zweite und/oder die dritte Ader typischerweise mit einer Energieversorgung, insbesondere einer Strom- und oder Spannungsquelle, des Steuergeräts verbunden. Es kann beispielsweise vorgesehen sein, dass das Steuergerät eine Aufnahme für einen Netzstecker, eine Batterie und/oder einen Akkumulator aufweist, über den das Steuergerät mit elektrischer Energie versorgbar ist. Über die erste, die zweite und/oder die dritte Ader ist auf diese Weise typischerweise auch die Herzpumpe mit elektrischer Energie versorgbar.

Die Versorgung der Herzpumpe mit elektrischer Energie kann beispielsweise für eine Versorgung von Sensoren in der Herzpumpe und/oder weiterer elektronischer Komponenten der Herzpumpe mit Gleichstrom und/oder mit Wechselstrom vorgesehen sein. Es kann zusätzlich oder alternativ vorgesehen sein, dass die erste Ader, die zweite Ader und/oder die dritte Ader mit einem Motor der Herzpumpe, insbesondere mit einem Stator des Motors, verbunden sind. Die Herzpumpe kann über die erste Ader, die zweite Ader und/oder die dritte Ader zum Fördern von Blut eines Patienten mit der elektrischen Energie, insbesondere mit Gleich- und/oder mit Wechselstrom, versorgbar sein.

In typischen Ausführungen ist das Steuergerät eingerichtet, die Herzpumpe derart zu versorgen, dass die erste, die zweite und die dritte Ader jeweils einen von drei zueinander phasenverschobenen Motorströmen führen. Der Motor der Herzpumpe kann beispielsweise als Synchronmotor ausgeführt sein. Durch eine Frequenz des Wechselstromes kann hierbei eine Drehfrequenz des Rotors mitsamt einer mit dem Rotor gegebenenfalls verbundenen Beschaufelung und somit eine geförderte Menge Blut festgelegt werden.

Das Trägerfrequenzsignal weist in typischen Ausführungen eine Frequenz von zumindest 1 MHz und/oder höchstens 10 MHz, beispielsweise 5 MHz oder 6 MHz, auf. Dieser Frequenzbereich eignet sich besonders dafür, Interferenzen zwischen den elektrischen Signalen für die Datenübertragung und Wechselströmen für die Energieübertragung zur Herzpumpe zu vermeiden.

Die Herzpumpe kann des Weiteren einen Ortsmodulator aufweisen. Es kann vorgesehen sein, dass der Ortsmodulator eingerichtet ist, ein Trägersignal und Daten zu empfangen. Ferner kann der Ortsmodulator eingerichtet sein, abhängig von diesen Daten ein Umschalten zwischen einem Einkoppeln des Trägersignals über die Koppelschnittstelle der Herzpumpe in eine von zwei der Adern, beispielsweise zwischen der ersten und der zweiten Ader oder zwischen der zweiten und der dritten Ader, zu bewirken. Durch den Ortsmodulator können somit binäre elektrische Signale an das Steuergerät gesendet werden. Der Ortsmodulator weist in der Regel einen elektronischen Schalter auf, durch den das Umschalten bewirkt wird. Beispielsweise koppelt der Ortsmodulator das Trägersignal in die zweite Ader ein, wenn der Ortsmodulator einen binären Wert "1" als Dateneingang erhält. Wenn der Ortsmodulator hingegen einen binären Wert "0" als Dateneingang erhält, kann der Ortsmodulator das Trägersignal in die dritte Ader einkoppeln. Auf diese Weise können die zweite Ader und die dritte Ader je ein zueinander invertiertes binäres Signal führen. Das Trägersignal kann dem Trägerfrequenzsignal entsprechen und/oder beispielsweise ein von dem Steuergerät gesendetes Signal sein, beispielsweise ein durch den Modulator des Steuergeräts moduliertes oder ein unmoduliertes Trägersignal. Wenn das Trägersignal ein von dem Modulator des Steuergeräts moduliertes, insbesondere ASK-moduliertes Signal ist, ist ein Modulationsgrad, d.h. ein Quotient aus einer Amplitude, die einem binären Wert 1 entspricht, und einer Differenz zwischen dieser Amplitude und einer Amplitude, die einem binären Wert "0" entspricht, typischerweise geringer als 1. Natürlich ist der Modulationsgrad in der Regel größer als "0". Durch das durch den Ortsmodulator bewirkte Umschalten werden die elektrischen Signale redundant auf zwei Adern von der Herzpumpe an das Steuergerät übertragen, so dass eine Plausibilitätsprüfung der von dem Steuergerät empfangenen Daten im Steuergerät erfolgen und eine Fehlererkennung im Steuergerät durchgeführt werden kann. Somit kann auf beschriebene Weise eine Störanfälligkeit der Datenübertragung reduziert werden.

Das Steuergerät und/oder die Herzpumpe können auch einen Wandler, insbesondere einen S2D(single-ended to differential)-Converter, umfassen. Ein solcher Wandler kann eingerichtet sein, invertierte Signale aus den an die Herzpumpe bzw. an das Steuergerät über eine der Adern zu übertragenden elektrischen Signalen zu erzeugen. Dieses nicht-invertierte Signal kann über die eine der Adern, beispielsweise die erste Ader, übertragen werden. Das Steuergerät bzw. die Herzpumpe ist dann typischerweise ebenfalls eingerichtet, die invertierten Signale in eine weitere der Adern, beispielsweise in die zweite Ader, einzukoppeln. Typischerweise wird dabei das nicht-invertierte elektrische Signal zeitgleich mit dem invertierten Signal übertragen. Die empfangende Herzpumpe bzw. das empfangende Steuergerät ist dann eingerichtet, die invertierten und die nicht-invertierten Signale aus den Adern auszukoppeln und differentiell auszuwerten, beispielsweise mittels eines Differenzverstärkers. Auf diese Weise kann eine Störungsempfindlichkeit der Datenübertragung reduziert werden, da sich gleichphasige Stör- oder Nutzsignale durch die differentielle Auswertung bei der empfangenden Herzpumpe bzw. beim empfangenden Steuergerät auslöschen. Dies kann besonders vorteilhaft sein, wenn auf einer oder mehreren der für die differentielle Übertragung genutzten Adern zeitgleich elektrische Signale in der Gegenrichtung übertragen werden. Es kann vorgesehen sein, dass ein Signal von einem Modulator wie oben beschrieben, insbesondere einem Amplitudenmodulator, dem (S2D-)Wandler zugeführt wird. Nach der differentiellen Auswertung kann ein resultierendes Signal einem Demodulator wie oben beschrieben, insbesondere einem Amplitudendemodulator, zugeführt werden.

Beispielsweise kann die Herzpumpe oder das Steuergerät in weiteren Ausführungen eingerichtet sein, an das Steuergerät beziehungsweise die Herzpumpe zu übertragende nicht-invertierte Signale gleichzeitig in die eine der Adern, beispielsweise in die erste Ader, und in die weitere der Adern, beispielsweise in die zweite Ader, einzukoppeln. Auf diese Weise wird das Signal gleichphasig in beiden Adern übertragen. Das gleichphasige Signal kann beim empfangenden Steuergerät bzw. bei der empfangenden Herzpumpe durch einen summierenden Verstärker ausgewertet werden. Auf diese Weise können gegebenenfalls auf den beiden Adern vorliegende gegenphasige Stör- oder auch Nutzsignale auf einfache Weise unterdrückt werden, so dass eine zuverlässige Datenübertragung gewährleistet ist. Es kann auf beiden Adern eine gegenphasige Signalübertragung auf einem Hinweg und eine gleichphasige Signalübertragung auf dem Rückweg erfolgen, so dass die Datenübertragung durch Auslöschung der gegenphasigen bzw. der gleichphasigen Signale am Empfänger besonders robust, d.h. wenig störanfällig, ist.

Somit ermöglichen die oben und unten beschriebenen Ausführungen auf eine einfache Weise eine besonders störungsarme Datenübertragung in zwei Richtungen insbesondere für den Fall, dass nur eine begrenzte Anzahl von Adern, beispielsweise zwei oder drei, zur Datenübertragung zur Verfügung steht. Wenn drei Adern zur Datenübertragung vorgesehen sind, können die elektrischen Signale in oben beschriebener Weise auf der ersten und zweiten Ader auf einem Hinweg differentiell und auf einem Rückweg als zwei gleichphasige elektrische Signale übertragen werden. Zudem kann über die dritte Ader auf dem Rückweg zusätzlich ein invertiertes der gleichphasigen elektrischen Signale übertragen werden. Die auf dem Rückweg übertragenen elektrischen Signale können durch die empfangende Herzpumpe bzw. das empfangende Steuergerät auch differentiell ausgewertet werden, beispielsweise mittels eines Differenzverstärkers, indem eine Differenz zwischen den nicht-invertierten und gleichphasigen elektrischen Signalen und dem invertierten Signal aus der dritten Ader ausgewertet wird. Ein Ausgang des summierenden Verstärkers kann dann an einen ersten Eingang dieses Differenzverstärkers geführt werden, wobei das invertierte Signal aus der dritten Ader zu einem zweiten Eingang dieses Differenzverstärkers geführt wird.

In Hinblick auf die Herzpumpeneinrichtung beschriebene Merkmale sind auf das Betriebsverfahren für die Herzpumpeneinrichtung übertragbar und umgekehrt.

Ausführungsbeispiele werden nachfolgend anhand der Abbildungen beschrieben. Es zeigt
- Fig. 1: eine schematische Ansicht einer Herzpumpeneinrichtung mit einer implantierten Herzpumpe und einem extrakorporalen Steuergerät,
- Fig. 2: eine schematische Ansicht der Herzpumpeneinrichtung,
- Fig. 3: eine schematische Ansicht einer Herzpumpeneinrichtung gemäß einem ersten Ausführungsbeispiel,
- Fig. 4: eine schematische Ansicht einer Herzpumpeneinrichtung gemäß einem zweiten Ausführungsbeispiel sowie
- Fig. 5: eine schematische Ansicht einer Herzpumpeneinrichtung gemäß einem dritten Ausführungsbeispiel.

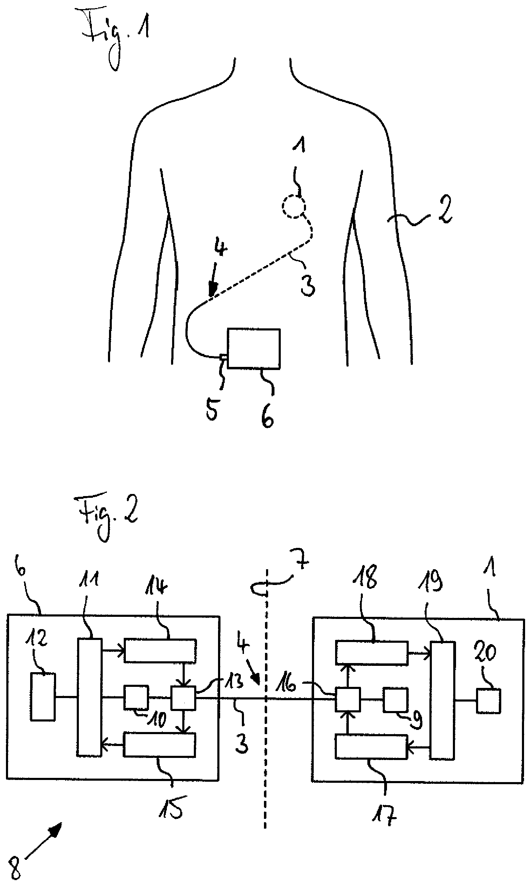

Figur 1 zeigt schematisch eine Ansicht einer Vorderseite eines Oberkörpers eines Patienten. Die Abbildung zeigt zudem eine Herzpumpe 1, die zur Unterstützung einer Herzfunktion des Patienten in dessen Körper 2 implantiert ist. Die Herzpumpe 1 weist einen in einem implantierbaren, biokompatiblen und fluiddicht verschweißten Pumpengehäuse angeordneten Motor mit einem antreibbaren Rotor auf. Die Herzpumpe 1 kann beispielsweise als sogenanntes LVAD-System ausgeführt und derart im Körper 2 des Patienten angeordnet sein, dass durch eine Drehung des Rotors Blut vom linken Herzventrikel in die Aorta förderbar ist.

Die Herzpumpe 1 ist mit einer transkutanen Driveline 3, die teilweise unter einer Haut des Patienten verläuft, verbunden. Außerhalb einer Einstichstelle 4 ist die Driveline 3 über einen Stecker 5 mit einem Steuergerät 6 verbunden. Das Steuergerät 6 versorgt die Herzpumpe 1 über die Driveline 3 mit elektrischer Energie.

Das Steuergerät 6 ist in typischen Ausführungen außen am Körper 2 des Patienten tragbar ausgeführt. Zu diesem Zweck kann das Steuergerät 6 zum Beispiel mit einer Halterung verbunden sein und über diese Halterung mit dem Körper 2 des Patienten verbunden sein. Beispielsweise kann die Halterung ein Gürtel, ein Tragegurt, eine Tragetasche oder eine Klebeverbindung sein, was in der Fig. 1 nicht dargestellt ist.

Figur 2 zeigt eine weitere schematische Ansicht der Herzpumpe 1, der Driveline 3 und des Steuergeräts 6. Wiederkehrende Merkmale sind in dieser Abbildung und in den nachfolgenden Abbildungen mit den gleichen Bezugszeichen versehen. Zudem ist schematisch eine Hautoberfläche 7 des Patienten dargestellt. Die Herzpumpe 1, die Driveline 3 und das Steuergerät 6 bilden in einigen Ausführungen eine Herzpumpeneinrichtung 8. Ein Steuergerät 6 wie oben oder unten beschrieben kann auch ohne die Herzpumpe 1 beansprucht werden. Zudem kann auch eine Herzpumpe 1 wie oben oder unten beschrieben ohne das Steuergerät 6 beansprucht werden.

Die Herzpumpe 1 umfasst den Motor 9, der in dem gezeigten Beispiel mit der Driveline 3 verbunden ist, so dass der Motor 9 von dem Steuergerät 6 zum Fördern von Blut des Patienten mit Energie versorgbar ist. Zu diesem Zweck umfasst der Motor 9 in der Regel einen Stator und den drehbar in dem Stator gelagerten Rotor. Der Rotor weist in der Regel eine Beschaufelung zum Fördern des Blutes auf. Wicklungen des Stators sind hierbei in der Regel zur Energieversorgung des Motors elektrisch mit der Driveline 3 verbunden, so dass die Wicklungen des Stators von dem Steuergerät 6 mit Strom versorgbar sind. Alternativ oder zusätzlich zu der Energieversorgung des Motors 9 kann es vorgesehen sein, dass weitere elektronische Komponenten der Herzpumpe 1 über die Driveline 3 mit elektrischer Energie versorgbar sind.

Auf Seiten des Steuergeräts 6 ist die Driveline 3 mit einem Motortreiber 10 verbunden. Der Motortreiber 10 ist in dem gezeigten Beispiel außerdem über einen Mikrocontroller 11 des Steuergeräts 6 mit einer Strom- bzw. Spannungsquelle 12 verbunden. Die Strom- bzw. Spannungsquelle 12 ist somit eingerichtet, die Herzpumpe 1, insbesondere den Motor 9 der Herzpumpe 1 und/oder die weiteren elektronischen Komponenten der Herzpumpe 1, über die Driveline 3 mit elektrischer Energie zu versorgen. Die Strom- bzw. Spannungsquelle 12 kann beispielsweise eine Batterie oder ein Akkumulator, insbesondere ein Steckakkumulator, sein oder durch eine Netzverbindung gebildet werden.

Das Steuergerät 6 und die Herzpumpe 1 sind zudem eingerichtet, Daten über die Driveline 3 miteinander auszutauschen. Die Driveline 3 umfasst in der Regel mehrere Adern, die von einem Mantel umschlossen sind. Die Datenübertragung erfolgt hierbei zumindest über eine Ader der Driveline 3, über die auch ein Strom für die oben beschriebene Energieversorgung der Herzpumpe 1 geführt wird. Zu diesem Zweck weist das Steuergerät 6 eine Koppelschnittstelle 13 auf, die über einen Modulator 14 und über einen Demodulator 15 mit dem Mikrocontroller 11 des Steuergeräts 6 verbunden ist. Entsprechend weist auch die Herzpumpe 1 eine Koppelschnittstelle 16 auf, die über einen Modulator 17 und einen Demodulator 18 mit einem Mikrocontroller 19 der Herzpumpe 1 verbunden ist. Die Koppelschnittstellen 13, 16 können in einigen Ausführungen jeweils zumindest einen Kondensator aufweisen, so dass die elektrischen Signale für die Datenübertragung kapazitiv in die Adern eingekoppelt werden (alternativ ist auch eine induktive Einkopplung möglich). Typischerweise weisen die Koppelschnittstellen 13, 16 zudem jeweils einen Hochpass oder einen Bandpass zur Unterdrückung niederfrequenter Motorstromsignale oder auch zur Unterdrückung niederfrequenter Störsignale auf.

In dem dargestellten Beispiel ist der Mikrocontroller 19 außerdem mit einem oder mehreren Sensoren verbunden, wie schematisch mit dem Bezugszeichen 20 illustriert ist. Über die Koppelschnittstelle 15 der Herzpumpe 1 können Daten, wie beispielsweise von dem Sensor 20 ermittelte Sensordaten oder Fehlerdaten, auf die Ader der Driveline 3 aufmoduliert, d.h. in diese Ader eingekoppelt werden, über die die Herzpumpe 1 gleichzeitig mit Energie versorgt wird. Diese Daten können über die Koppelschnittstelle 13 des Steuergeräts 6 aus der Ader ausgekoppelt, durch den Demodulator 15 demoduliert und an den Mikrokontroller 11 des Steuergeräts 6 übertragen werden. Entsprechend können beispielsweise Daten zu Betriebs- oder Steuerparametern des Motors 9 von dem Mikrocontroller 11 des Steuergeräts 6 an den Mikrocontroller 19 der Herzpumpe 1 übertragen werden.

Während in dem dargestellten Beispiel eine Energieversorgung des Motors 9 durch die Strom- bzw. Spannungsquelle 12 gezeigt ist, ist es zusätzlich oder alternativ natürlich auch möglich, dass beispielsweise der Mikrocontroller 19 der Herzpumpe 1 und/oder die Sensoren 20 in beschriebener Weise über die Driveline 3 durch die Strom- bzw. Spannungsquelle 12 mit elektrischer Energie versorgt werden.

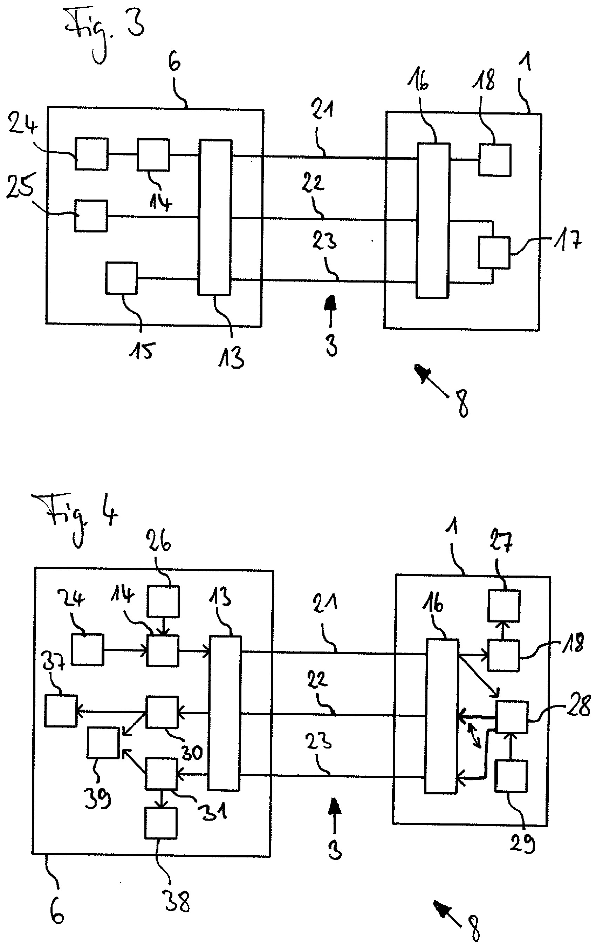

Eine mögliche Ausgestaltung der Herzpumpeneinrichtung 8, bei der die Daten durch eine Aufmodulation von elektrischen Signalen auf mehrere Adern der Driveline 3 übertragen werden, zeigt Figur 3. Hierbei werden die Signale über eine oder mehrere Trägerfrequenzen zusätzlich zu der elektrischen Energieversorgung der Herzpumpe 1 auf die Adern moduliert. Die Driveline 3 weist in dem gezeigten Beispiel drei Adern, d.h. eine erste 21, eine zweite 22 sowie eine dritte Ader 23, auf. Über diese Adern 21, 22, 23 wird die Herzpumpe 1 mit elektrischer Energie versorgt. In einigen Ausführungen können diese Adern 21, 22, 23 mit dem Stator des Motors 9 verbunden sein, zueinander phasenverschobenen Wechselstrom führen und somit drei Motorphasen zum Antrieb des Motors 9 und zur Förderung des Bluts des Patienten bilden. Zur Datenübertragung weist das Steuergerät 6 einen ersten Oszillator 24 zur Erzeugung einer ersten Trägerfrequenz sowie einen zweiten Oszillator 25 zur Erzeugung einer zweiten Trägerfrequenz auf. Die Trägerfrequenzen können beispielsweise in Form eines sinusförmigen Trägersignals erzeugt werden.

In diesem Ausführungsbeispiel werden Daten von dem Steuergerät 6 an die Herzpumpe 1 über die erste Ader 21 und Daten von der Herzpumpe 1 an das Steuergerät 6 über die dritte Ader 23 übertragen. Somit sind zwei im Wesentlichen voneinander unabhängige Systeme für beide Übertragungsrichtungen vorgesehen. Jedes dieser Systeme besteht aus einer Sendeeinheit, welche die zu übertragenden Daten auf eines der Trägersignale aufmoduliert, und einer entsprechenden Empfangseinheit, die das Signal demoduliert und so die zu empfangenden Daten ausgibt. Für die Modulation sind grundsätzlich an sich bekannte Modulationsarten verwendbar; beispielsweise kann für beide Übertragungsrichtungen eine ASK-Modulation vorgesehen sein, da diese sowohl beim Modulieren als auch beim Demodulieren mit sehr geringem Bauteilaufwand umgesetzt werden kann. Zur Datenübertragung von dem Steuergerät 6 zu der Herzpumpe 1 wird die erste Trägerfrequenz durch den Modulator 14 des Steuergeräts 6 entsprechend den zu sendenden Daten moduliert und über die Koppelschnittstelle 13 des Steuergeräts 6 und die erste Ader 21 an die Herzpumpe 1 übertragen. Hier wird dieses modulierte Signal durch die Koppelschnittstelle 16 der Herzpumpe 1 aus der ersten Ader 21 ausgekoppelt und durch den Demodulator 18 der Herzpumpe 1 demoduliert, so dass diese Daten durch den Mikrocontroller 19 der Herzpumpe 1 ausgewertet werden können. Für eine Dekodierung am Empfänger kann eine Synchronisierung mit einer Taktrate des Senders erfolgen, insbesondere bei synchroner Datenübertragung. Die Übertragung des Datentaktes kann über Taktrückgewinnungsmethoden aus dem Datenstrom extrahiert werden, es kann aber auch vorgesehen sein, dass der Datentakt explizit übertragen wird. Es kann insbesondere bei asynchronen Kommunikationspfaden auch vorgesehen sein, dass eine Abstimmung durch das Übersenden von Synchronisationsnachrichten, beispielsweise Synch-Bytes, erfolgt.

Um den Bauteilaufwand in der Herzpumpe 1 möglichst gering zu halten, wird das zweite Trägersignal für die Übertragungsrichtung von der Herzpumpe 1 zu dem Steuergerät 6 in dem Steuergerät 6 durch den zweiten Oszillator 25 erzeugt und über die Koppelschnittstelle 13 des Steuergeräts 6 zur Herzpumpe 1 übertragen. Auf Seiten der Herzpumpe 6 wird das zweite Trägersignal anschließend mit dem Modulator 17 der Herzpumpe 1 moduliert. Dieses modulierte Signal wird anschließend zur Datenübertragung an das Steuergerät 6 übertragen und durch den Demodulator 15 des Steuergeräts 6 demoduliert, so dass der Mikrocontroller 11 des Steuergeräts 6 diese Daten auswerten kann. Zur Amplitudenmodulation der Trägerfrequenzen können die Modulatoren 14, 17 beispielsweise jeweils einen Spannungsteiler aufweisen, durch den eine Amplitude der Trägerfrequenz je nach einem empfangenen binären Datenwert moduliert wird. In der beschriebenen Ausführung werden zwei Übertragungsmedien für die Übertragung der Daten von der Herzpumpe 1 zum Steuergerät 6 benötigt. Folglich werden hierfür zwei der drei Adern bzw. Motorleitungen 22, 23 verwendet. Die dritte vorhandene Ader 23 wird dann für die andere Übertragungsrichtung verwendet.

Die zur Datenübertragung verwendeten elektrischen Signale (sowohl moduliert als auch nicht moduliert) können hochfrequente Signale sein. Typischerweise ist eine Frequenz dieser Signale größer als eine Wechselstromfrequenz, die für den Antrieb des Motors über die Adern 21, 22, 23 übertragen wird. Es kann vorgesehen sein, dass für beide Übertragungsrichtungen die gleiche Trägerfrequenz verwendet wird. Um durch kapazitive Kopplungen zwischen den Adern hervorgerufene Störungen zu vermeiden, kann es jedoch auch vorgesehen sein, dass sich die erste Trägerfrequenz von der zweiten Trägerfrequenz unterscheidet. Beispielsweise kann eine Frequenz des ersten Trägersignals 5 MHz betragen, während eine Frequenz des zweiten Trägersignals 6 MHz beträgt.

Ein weiteres Ausführungsbeispiel der Herzpumpeneinrichtung 8 ist schematisch in Figur 4 gezeigt. In dieser Ausführung wird wie in dem oben beschriebenen Beispiel keine Trägerfrequenz in der typischerweise platzsparend ausgeführten Herzpumpe 1 erzeugt. Gemäß dieser Ausführung ist hingegen lediglich ein einzelner Oszillator 24 zur Erzeugung einer Trägerfrequenz notwendig, wobei das von diesem Oszillator 24 erzeugte Trägerfrequenzsignal für beide Übertragungsrichtungen verwendet wird. Dieser Oszillator 24 ist als Bestandteil des Steuergeräts 6 ausgeführt und kann wie oben beschrieben ausgebildet sein.

Die Datenübertragung von dem Steuergerät 6 an die Herzpumpe 1 kann in diesem Beispiel wie oben beschrieben erfolgen, wobei an die Herzpumpe 1 zu übertragende Daten über einen Dateneingang 26 des Steuergeräts 6 dem Modulator 14 des Steuergeräts 6 zugeführt und als modulierte Signale über die erste Ader 21 an den Demodulator 18 der Herzpumpe 1 übermittelt und weiterhin an einen Datenausgang 27 der Herzpumpe 1 übertragen werden. Hierbei kann das Signal amplitudenmoduliert übertragen werden, wobei ein Modulationsgrad kleiner als eins zu wählen ist, so dass das an die Herzpumpe 1 übertragene Signal zu keinem Zeitpunkt die Amplitude null aufweist und wie unten beschrieben als Trägersignal für die umgekehrte Übertragungsrichtung weiterverwendet werden kann.

Zur Übertragung von Daten von der Herzpumpe 1 an das Steuergerät 6 weist die Herzpumpe 1 einen Ortsmodulator 28 auf. Der Ortsmodulator 28 ist mit der Koppelschnittstelle 16 der Herzpumpe 1 und mit einem Dateneingang 29 der Herzpumpe 1 verbunden. Neben den von dem Dateneingang 29 empfangenen und an das Steuergerät 6 zu übertragenden binären Daten empfängt der Ortsmodulator 28 von der Koppelschnittstelle 16 der Herzpumpe 1 das über die erste Ader 21 übertragene modulierte Trägersignal. Je nach empfangenem Datenwert überträgt der Ortsmodulator 28 das modulierte Trägersignal auf einer der beiden anderen Adern 22, 23 zurück zum Steuergerät 6 und schaltet somit je nach empfangenem Datenwert zwischen einem Einkoppeln in die zweite Ader 22 beziehungsweise in die dritte Ader 23 um. Empfängt der Ortsmodulator 28 beispielsweise eine logische "1" am Dateneingang 29, dann kann dieser beispielsweise das empfangene Trägersignal in die zweite Ader 22 weiterleiten. Empfängt der Ortsmodulator 28 hingegen eine logische "0" am Dateneingang 29, dann kann dieser beispielsweise das empfangene Trägersignal in die dritte Ader 23 weiterleiten.

Zur Demodulation der von dem Ortsmodulator 28 modulierten elektrischen Signale auf Seiten des Steuergeräts 6 können die über die beiden Adern 22, 23 empfangenen Signale amplitudendemoduliert werden. Zu diesem Zweck weist das Steuergerät 6 einen ersten Amplitudendemodulator 30 und einen zweiten Amplitudendemodulator 31 auf. Der erste Amplitudendemodulator 30 gibt dabei die zu empfangenen binären Daten aus, was schematisch mit dem Bezugszeichen 37 gezeigt ist. Der zweite Amplitudendemodulator 31 gibt hingegen eine Inversion dieser Daten aus, was schematisch durch das Bezugszeichen 38 dargestellt ist. Es kann zudem vorgesehen sein, dass die von den Amplitudendemodulatoren 30, 31 ausgegebenen Signale anschließend einem Differenzverstärker 39 zur differentiellen Auswertung der von dem Steuergerät 6 empfangenen Daten zugeführt werden. Hierbei können Hüllkurven der über die beiden Adern 22, 23 empfangenen Signale voneinander subtrahiert werden, so dass sich auf diese Weise das gewünschte Nutzsignal ergibt.

Das Steuergerät 6 kann zudem eine nicht dargestellte Entscheidungslogik aufweisen, der die von den Amplitudendemodulatoren 30, 31 ausgegebenen Signale zugeführt werden. Auf diese Weise können bei der Demodulation oder bei der Übertragung möglicherweise auftretende Fehler erkannt werden. Es kann durch die beschriebene Übertragung auf zwei Adern 22, 23 und anschließende jeweilige Demodulation zudem ein Ausfall eines der Amplitudendemodulatoren 30, 31 kompensiert werden.

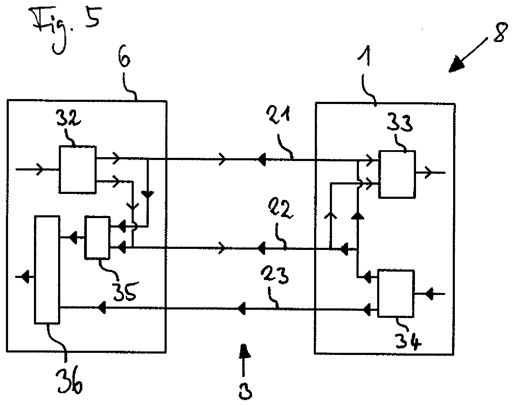

Ein weiteres Ausführungsbeispiel der Herzpumpeneinrichtung 8 zeigt Figur 5. Diese Ausführung unterscheidet sich von den oben beschriebenen Ausführungen darin, dass beide Übertragungsrichtungen differentiell aufgebaut sind. Zunächst wird wie oben beschrieben ein Trägersignal in dem Steuergerät 6 erzeugt und anschließend zur Datenübertragung an die Herzpumpe 1 moduliert, insbesondere amplitudenmoduliert. Das modulierte Signal wird anschließend über einen S2D-Wandler 32 (single-ended to differential converter) in ein differentielles Signal umgewandelt. Dieses differentielle Signal wird in zwei der drei Adern 21, 22 eingekoppelt, derart, dass die erste Ader 21 das modulierte Signal und die zweite Ader 22 ein dazu invertiertes Signal führt. Auf Seiten der Herzpumpe 1 werden diese Signale ausgekoppelt und differentiell ausgewertet. Hierfür können die Signale beispielsweise mit einem Differenzverstärker 33 oder einem differentiellen Demodulator ausgewertet bzw. demoduliert werden.

Für die Datenübertragung von der Herzpumpe 1 zu dem Steuergerät 6 wird in der Herzpumpe 1 mit einem Oszillator ein weiteres Trägersignal erzeugt und moduliert, insbesondere amplitudenmoduliert. Eine Frequenz dieses Trägersignals unterscheidet sich hierbei von einer Frequenz des Trägersignals, welches in dem Steuergerät 6 erzeugt wird. Das modulierte Signal wird über einen S2D-Wandler 34 der Herzpumpe 1 umgewandelt, zum Steuergerät 6 übertragen und schließlich ähnlich wie oben beschrieben mit einem Differenzverstärker 36 des Steuergeräts 6 differentiell ausgewertet. Da für zwei differentielle Übertragungsrichtungen vier Übertragungskanäle benötigt werden und in dem gezeigten Beispiel lediglich drei das Steuergerät 6 mit der Herzpumpe 1 verbindende Adern 21, 22, 23 vorhanden sind, muss zumindest eine Ader für eine Signalübertragung in zwei Richtungen genutzt werden. In dem gezeigten Ausführungsbeispiel werden sowohl die erste Ader 21 als auch die zweite Ader 22 für eine bidirektionale Signalübertragung genutzt. Um hierbei Störungen zu vermeiden, ist die Koppelschnittstelle 16 der Herzpumpe 1 eingerichtet, von dem S2D-Wandler 34 der Herzpumpe 1 gelieferte Signale gleichphasig in die erste Ader 21 und die zweite Ader 22 einzukoppeln, so dass diese Signale redundant übertragen werden. Zudem liefert der S2D-Wandler 34 der Herzpumpe 1 ein invertiertes Signal an die Koppelschnittstelle 16, das diese in die dritte Ader 23 zur Übertragung an das Steuergerät 6 einkoppelt.

Die über die erste Ader 21 und die zweite Ader 22 an das Steuergerät 6 übertragenen Signale werden durch die Koppelschnittstelle 13 des Steuergeräts 6 aus den Adern 21, 22 ausgekoppelt und einem summierenden Verstärker 35 des Steuergeräts 6 zugeführt. Ein Ausgangssignal dieses summierenden Verstärkers 35 wird anschließend zur differentiellen Auswertung an den Differenzverstärker 36 des Steuergeräts 6 geleitet, der außerdem das aus der dritten Ader 23 ausgekoppelte invertierte Signal erhält. In einem weiteren Schritt kann das von dem Differenzverstärker 35 ausgegebene Signal demoduliert werden, so dass die Daten vollständig an das Steuergerät 1 übertragen sind. Indem die auf der ersten Ader 21 und der zweiten Ader 22 an die Herzpumpe 1 übertragenen elektrischen Signale gegenphasig (invers oder gegentaktig) und die auf diesen Adern 21, 22 an das Steuergerät übertragenen Daten gleichphasig (nicht-invertiert oder gleichtaktig) sind, kann durch eine differentielle Auswertung auf Seiten der Herzpumpe 1 bzw. eine summierende Auswertung auf Seiten des Steuergeräts 6 eine störende gegenseitige Beeinflussung der auf diesen Adern 21, 22 übertragenen elektrischen Signale verhindert werden.

Lediglich in den Ausführungsbeispielen offenbarte Merkmale der verschiedenen Ausführungsformen können miteinander kombiniert und einzeln beansprucht werden.

## Patentansprüche

1. Herzpumpeneinrichtung (8), umfassend eine implantierbare Herzpumpe (1) und ein Steuergerät (6) zum Steuern der Herzpumpe (1), wobei das Steuergerät (6) und die Herzpumpe (1) über eine Leitung mit Adern (21, 22, 23) miteinander verbunden sind, wobei das Steuergerät (6) eingerichtet ist, die Herzpumpe (1) über eine erste der Adern (21) mit elektrischer Energie zu versorgen,
wobei das Steuergerät (6) und die Herzpumpe (1) jeweils eine Koppelschnittstelle (13, 16) aufweisen, wobei über diese Koppelschnittstellen (13, 16) elektrische Signale zur Datenübertragung zwischen dem Steuergerät (6) und der Herzpumpe (1) in die erste Ader (21) einkoppelbar beziehungsweise aus der ersten Ader (21) auskoppelbar sind.

2. Herzpumpeneinrichtung (8) nach Anspruch 1, wobei die Leitung eine zweite Ader (22) aufweist, wobei die Herzpumpe (1) über die erste Ader (21) und die zweite Ader (22) mit elektrischer Energie versorgbar ist, wobei über die Koppelschnittstellen (13, 16) des Steuergeräts (6) und der Herzpumpe (1) weitere elektrische Signale zur Datenübertragung zwischen dem Steuergerät (6) und der Herzpumpe (1) in die zweite Ader (22) einkoppelbar beziehungsweise aus der zweiten Ader (22) auskoppelbar sind.

3. Herzpumpeneinrichtung (8) nach einem der vorangegangenen Ansprüche, wobei das Steuergerät (6) und die Herzpumpe (1) zum Übertragen von Daten von der Herzpumpe (1) zu dem Steuergerät (6) über die Koppelschnittstellen (13, 16) und über die erste und/oder die zweite Ader (21, 22) eingerichtet sind.

4. Herzpumpeneinrichtung (8) nach einem der vorangegangenen Ansprüche, wobei das Steuergerät (6) und die Herzpumpe (1) zum Übertragen von Daten von dem Steuergerät (6) zu der Herzpumpe (1) über die Koppelschnittstellen (13, 16) und über die erste und/oder die zweite Ader (21, 22) eingerichtet sind.

5. Herzpumpeneinrichtung (8) nach einem der vorangegangenen Ansprüche, wobei das Steuergerät (6) einen mit der Koppelschnittstelle (13) des Steuergeräts (6) verbundenen Modulator (14) aufweist, der eingerichtet ist, ein Trägerfrequenzsignal zum Erzeugen der in die erste und/oder die zweite Ader (21, 22) einzukoppelnden elektrischen Signale zur Datenübertragung an die Herzpumpe (1) zu modulieren.

6. Herzpumpeneinrichtung (8) nach einem der vorangegangenen Ansprüche, wobei die Herzpumpe (1) einen mit der Koppelschnittstelle (16) der Herzpumpe (1) verbundenen Modulator (17) aufweist, der eingerichtet ist, ein Trägerfrequenzsignal zum Erzeugen der in die erste und/oder die zweite Ader (21, 22) einzukoppelnden elektrischen Signale zur Datenübertragung an das Steuergerät (6) zu modulieren.

7. Herzpumpeneinrichtung (8) nach Anspruch 6, sofern dieser auf Anspruch 2 rückbezogen ist, wobei das Steuergerät (6) eingerichtet ist, ein Trägerfrequenzsignal über die Koppelschnittstelle (13) des Steuergeräts (6) in eine Ader (22) der Leitung einzukoppeln, wobei die Herzpumpe (1) eingerichtet ist, das Trägerfrequenzsignal über die Koppelschnittstelle (16) der Herzpumpe (1) auszukoppeln, und wobei der Modulator (17) der Herzpumpe (1) eingerichtet ist, das Trägerfrequenzsignal zum Erzeugen der in die erste und/oder die zweite Ader (21, 22) einzukoppelnden elektrischen Signale zur Datenübertragung an die Herzpumpe (1) zu modulieren.

8. Herzpumpeneinrichtung (8) nach einem der vorangegangenen Ansprüche, wobei die Herzpumpe (1) einen Ortsmodulator (28) aufweist, der eingerichtet ist, ein Trägersignal und Daten zu empfangen und abhängig von diesen Daten ein Umschalten zwischen einem Einkoppeln des Trägersignals über die Koppelschnittstelle (16) der Herzpumpe (1) in eine von zwei der Adern (22, 23) zu bewirken.

9. Herzpumpeneinrichtung (8) nach einem der vorangehenden Ansprüche, wobei die erste Ader (21) und/oder die zweite Ader (22) mit einem Motor (9) der Herzpumpe (1), insbesondere mit einem Stator des Motors (9), verbunden ist, wobei die Herzpumpe (1) über die erste Ader (21) und/oder die zweite Ader (22) zum Fördern von Blut eines Patienten mit der elektrischen Energie, insbesondere mit Wechselstrom, versorgbar ist.

10. Herzpumpeneinrichtung (8) nach Anspruch 9, wobei das Steuergerät (6) eingerichtet ist, die Herzpumpe (1) derart zu versorgen, dass die erste Ader (21), die zweite Ader (22) und eine dritte Ader (23) jeweils einen von drei zueinander phasenverschobenen Motorströmen führen.

11. Herzpumpeneinrichtung (8) nach einem der vorangegangenen Ansprüche, wobei das Steuergerät (6) oder die Herzpumpe (1) einen Wandler (32, 34), insbesondere einen S2D-Converter, umfasst, der eingerichtet ist, invertierte Signale aus den an die Herzpumpe (1) bzw. an das Steuergerät (6) über die erste Ader (21) zu übertragenden elektrischen Signalen zu erzeugen, wobei das Steuergerät (6) oder die Herzpumpe (1) eingerichtet ist, die invertierten Signale in die zweite Ader (22) einzukoppeln.

12. Herzpumpeneinrichtung (8) nach Anspruch 11, wobei die Herzpumpe (1) oder das Steuergerät (6) eingerichtet ist, an das Steuergerät (6) beziehungsweise die Herzpumpe (1) zu übertragende Signale gleichzeitig in die erste Ader (21) und in die zweite Ader (22) einzukoppeln.

13. Herzpumpeneinrichtung (8) nach einem der vorangegangenen Ansprüche, wobei die über die elektrischen Signale zwischen dem Steuergerät (6) und der Herzpumpe (1) ausgetauschten Daten Sensordaten, Patientendaten, Patientenidentifizierungsdaten, Hardwareidentifizierungsdaten, Softwareversionsdaten, Konfigurationsdaten, Betriebsparameter, Fehlerdaten, Informationen zu Warnbedingungen, Messdaten, Zeitinformationen, Rechenergebnisse, Informationen zur Rotorposition und/oder Motordaten sind.

14. Herzpumpeneinrichtung (8) nach einem der vorangegangenen Ansprüche, wobei das Trägerfrequenzsignal eine Frequenz von zumindest 1 MHz und/oder höchstens 10 MHz aufweist.

15. Betriebsverfahren für eine Herzpumpeneinrichtung (8) nach einem der vorangehenden Ansprüche, umfassend folgende Schritte:
- Übertragen von elektrischer Energie von dem Steuergerät (6) zu der Herzpumpe (1) über die erste Ader (21),
- Einkoppeln eines elektrischen Signals in die erste Ader (21) durch die Koppelschnittstelle (13) des Steuergeräts (6) oder die Koppelschnittstelle (16) der Herzpumpe (1) zum Übertragen von Daten zwischen dem Steuergerät (6) und der Herzpumpe (1) und
- Auskoppeln des elektrischen Signals aus der ersten Ader (21) durch die Koppelschnittstelle (16) der Herzpumpe (1) bzw. die Koppelschnittstelle (13) des Steuergeräts (6).
